# EUROPEAN PATENT SPECIFICATION

(11) **EP 0 260 137 B1**
(45) Date of publication and mention of the grant of the patent: **02.03.1994**
(21) Application number: 87308005.5
(22) Date of filing: 10.09.1987
(51) Int. Cl.: C12N 9/10, C12N 1/20, C12Q 1/00, C12Q 1/48, C12Q 1/34, C12Q 1/32, C12N 9/00

(54) **Assay method using novel NAD synthetase and a process for production of the enzyme**
Eine neue NAD-Synthetase benutzende Testmethode und Verfahren zur Herstellung des Enzyms
Méthode d'essai utilisant une nad synthétase et un procédé pour la production de l'enzyme

(30) Priority: 10.09.1986 JP 213679/86; 30.07.1987 JP 191018/87
(43) Date of publication of application: 16.03.1988
(60) Divisional application: 92118167.3
(73) Proprietor: ASAHI KASEI KOGYO KABUSHIKI KAISHA, Osaka (JP)
(72) Inventor: Takahashi, Mamoru, Sunto-gun Shizuoka-ken (JP); Misaki, Hideo, Tagata-gun Shizuoka-ken (JP); Imamura, Shigeyuki, Tagata-gun Shizuoka-ken (JP); Matsuura, Kazuo, Tagata-gun Shizuoka-ken (JP)
(74) Representative: Woods, Geoffrey Corlett

(56) References cited:
- FR-A- 2 545 504
- EXPERIENTIA, vol. 29, no. 8, 15th August 1973, pages 941-942; H. HENGARTNER et al.: "Isolation of different thermophilic enzymes from Bacillus stearothermophilus"
- THE JOURNAL OF BIOLOGICAL CHEMISTRY, vol. 247, no. 15, 10th August 1972, pages 4794-4802, US; C.K. YU et al.: "Purification and properties of yeast nicotinamide adenine dinucleotide synthetase"
- THE JOURNAL OF BIOLIGICAL CHEMISTRY, vol. 242, no. 3, 10th February 1967, pages 385-392, US; R.L. SPENCER et al.: "Biosynthesis of diphosphopyridine nucleotide"
- THE JOURNAL OF BIOLOGICAL CHEMISTRY, vol. 236, no. 5, May 1961, pages 1494-1497, US; J. IMSANDE: "Pathway of diphosphopyridine nucleotide biosynthesis in Escherichia coli"

## Description

This invention relates to a NAD synthetase, to a process for the production thereof and to a microorganism which may be used in the process.

More particularly, the present invention relates to a NAD synthetase ( hereinafter sometimes referred to as the present NAD synthetase ) which at least catalyses the reaction ( a ) as defined hereinbelow in the presence of Mg⁺⁺ or Mn⁺⁺ ions and which does not catalyse the reaction ( b ) as defined hereinbelow in the presence of Mg⁺⁺ ions, utilizes ammonia including ammonium ions, and does not utilize glutamine and asparagine, to a process for the production thereof, and to a method of assaying a specimen for ATP, deamide-NAD and ammonia including ammonia in the form of an ammonium ion, by incubating the specimen with the present NAD synthetase. 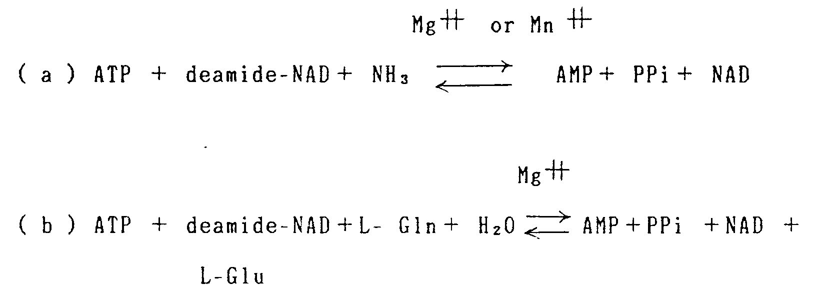
NAD synthetases are known to exist in rat liver [ J. Biol. Chem. 233 , 493 - 500 ( 1958 )] , porcine liver [ ibid., 236 , 525 - 530 (1961) ] , yeast [ ibid., 247 , 4794 - 4802 ( 1972 )] and E. Coli [ ibid., 236 , 1494 - 1497 ( 1961 ) and 242, 385 - 392 ( 1967 )] .

Said NAD synthetases are classified as NAD synthetase ( EC 6.3.1.5 ) which catalyses the reaction : 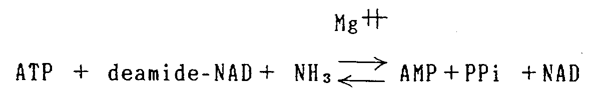
and NAD synthetase ( EC 6.3.5.1 ) which catalyses the reaction : 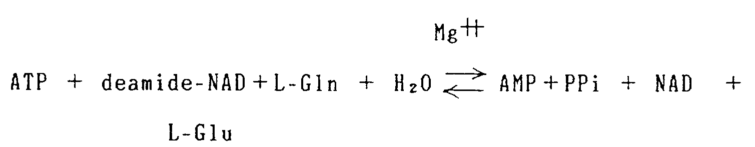
These NAD synthetases utilize NH₃ or an amide of L-Gln, and are differentiated by the inhibitory action of azaserine.

The km-value of NAD synthetase ( EC 6.3.1.5 ) is 6.5 x 10⁻⁵ M ( NH₄⁺ ) and 1.6 x 10 ⁻² M ( L-Gln ) [ J. Biol., Chem., 1494 - 1497, ( 1961 ), ibid., 242, 385 - 392 ( 1967) ] and the Km-value of NAD synthetase ( EC 6.3.5.1 ) is 6.4 x 10 ⁻⁸ M ( NH₄⁺ ) and 5 x 10 ⁻⁸ M ( L-Gln ) [ J. Biol. Chem. 247, 4794 - 4802 ( 1972 ), ibid. 233, 493 - 500 (1958) ] .

An assay method for NAD synthtase has been reported, in which the NAD generated is reduced by alcohol dehydrogenase ( EC 1.1.1 ) and the absorbancy of the generated and reduced NAD ( hereinafter referred to as NADH ) is spectorophotometrically measured at 340 nm or the amount of generated NAD is measured by fluorometry.

We have reported an assay method for a component in a specimen, said component being ATP, deamide-NAD or an amide donor such as NH₃, L-glutamine or L-asparagine, which comprises, as a main reaction step, incubating the specimen with the known NAD synthetase ( EC 6.3.1.5 or EC 6.3.5.1 ) in the presence of ATP, deamide-NAD, an amide donor and Mg⁺⁺ to generate NAD, performing a coenzyme cycling reaction by combining the oxidation-reduction reaction system with coenzyme NAD and the oxidation-reduction reaction system with coenzyme reduced NAD, and measuring the amount of a component consumed or generated in the cycling reaction ( JP-A-59-198995 ).

As explained, the known NAD synthetase utilizes the amide donor substrate L-glutamine. A NAD synthetase which at least catalyses the reactions ( a ) as defined above in the presence of Mg⁺⁺ or Mn⁺⁺ ions and does not catalyse the reaction ( b ) as defined above in the presence of Mg⁺⁺ ions, utilizes ammonia including ammonium ions and does not utilize glutamine and asparagine, is not known. Furthermore the known NAD synthetase has substrate specificity for L-glutamine, and so NH₃ cannot be measured in the presence of L-glutamine.

We have found that Bacillus sp. H-804, isolated from a soil sample obtained from hot spring water at Tanoyu-machi, Beppu-shi, Oita-ken, Japan, produces the present NAD synthetase.

The present invention provides a NAD synthetase having the following properties:
(1) Action:
   at least catalyses in the presence of Mg⁺⁺ or Mn⁺⁺ ion the reaction (a): 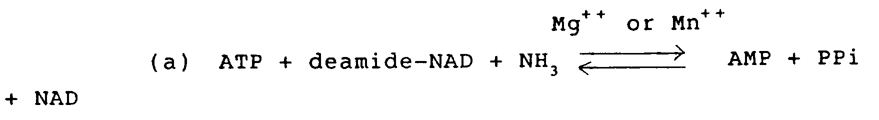 and does not catalyse in the presence of Mg⁺⁺ ion the reaction (b): 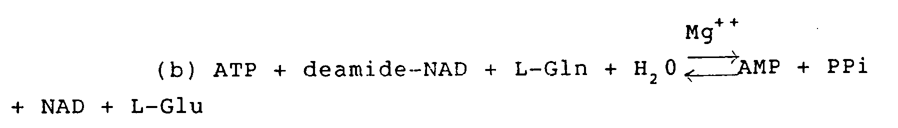
(2) Substrate specificity:
   utilises ammonia, including ammonia in the form of an ammonium ion, as a substrate but does not utilise glutamine or asparagine as a substrate;
(3) optimum pH : pH 8.5. - 10.0;
(4) pH-stability : pH 7.5 - 9.0 (at 60°, 15 min);
(5) optimum temperature : approx. 60°C;
(6) heat stability : below 60°C (at pH 8.0, 15 min);
(7) isoelectric point : approx. pH 5.3; and
(8) molecular weight : 50,000 ± 5,000 (molecular sieve using polyvinyl gel).

The present invention also provides a process for the preparation of a NAD synthetase as defined above, which process comprises culturing Bacillus stearothermophilus H-804 (FERM BP-1408) or a mutant thereof which is capable of producing said NAD synthetase and isolating the NAD synthetase thus-produced.

The present invention further provides Bacillus stearothermophilus H-804 (FERM BP-1408) or a mutant thereof which is capable of producing the NAD synthetase defined above.

The present invention additionally provides a culture of Bacillus stearothermophilus H-804 (FERM BP-1408) or a mutant thereof which is capable of producing the NAD synthetase defined above, in a culture medium which comprises a source of assimilable carbon, a source of assimilable nitrogen and mineral salts and which is free of other microorganisms.

The drawings illustrate various properties of the present invention and are explained in detail in the Examples and in the passage describing the properties of the present NAD synthetase. However a brief explanation of each Figure is given below:
- Fig. 1 :: Optimum pH of NAD synthetase;
- Fig. 2 :: pH-stability of NAD synthetase;
- Fig. 3 :: Heat-stability of NAD synthetase;
- Fig. 4 :: Optimum temperature of NAD synthetase;
The taxonomical properties of strain H-804 which produces the present NAD synthetase are as follows:

### A. Morphological properties:

Observed by microscope on nutrient agar slant medium at 50°C for 1 to 3 days cultivation.
- 1. Form and arrangement:: Round edges, straight or slightly curved bacilli, single or binary chains.
- 2. Size :: 0.5 to 1.0 x 1.6 to 5.5 µm.
- 3. Motility :: motile by peritrichous flagella.
- 4. Spores :: forms center or subterminal, 0.8 to 1.0 x 1.0 to 1.5 µm, swelling the sporangia.

### B. Growth on various media ( at 50°C ):

- 1. Nutrient agar plate :: Colonies grayish to pale yellowish white and semi-transparent, linear growth. weak growth. No soluble pigment formation.
- 2. Nutrient agar slant :: Grayish to pale yellowish and semi-transparent, round plain colonies. No solube pigment formation.
- 3. Bouillon agar :: Uniformly turbid, good growth. 2 to 3 days forms precipitation.
- 4. BCP milk :: No changes.

### C. Physiological properties ( +: positive, -: negative):

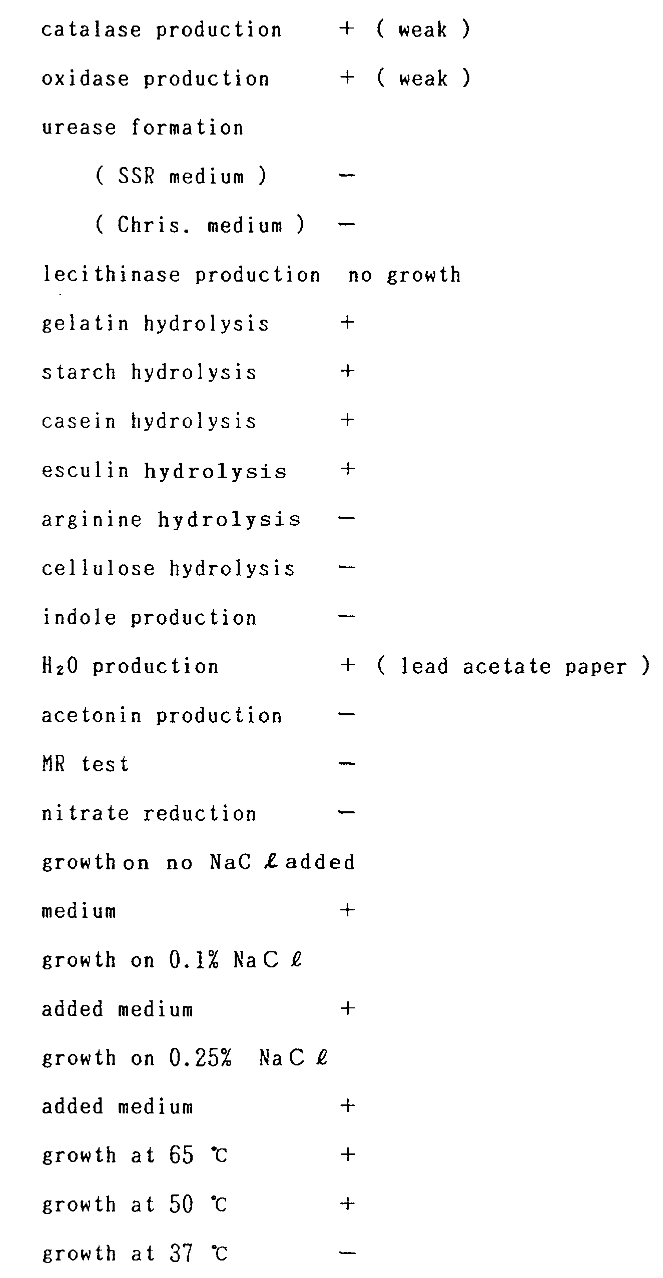

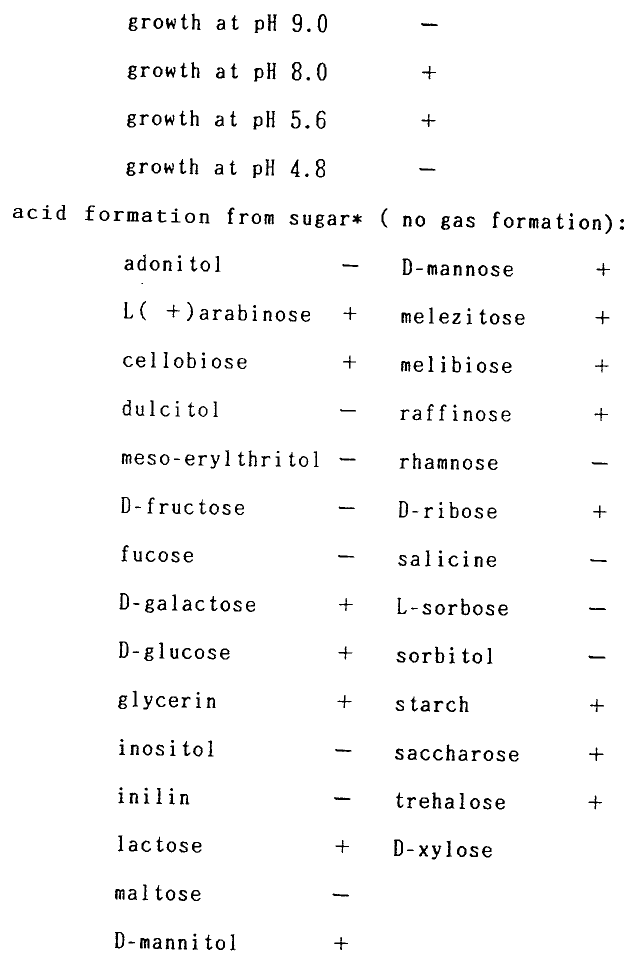

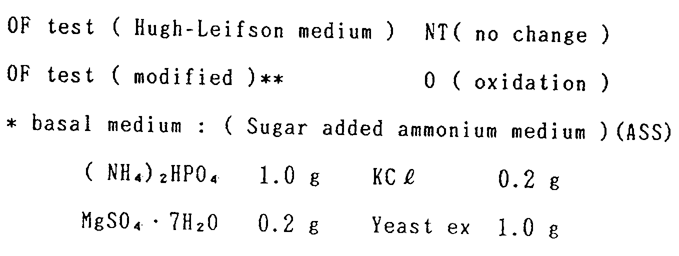

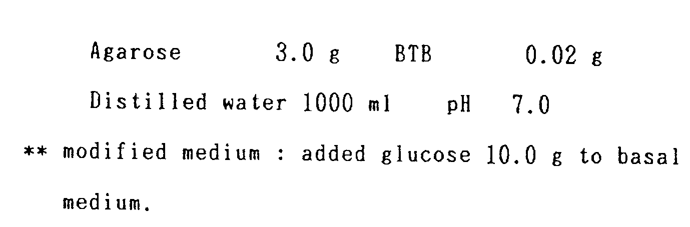

| Utilization test : | | |
|---|---|---|
| | Simmons medium | Christensen medium |
| citrate | - | - |
| malonate | - | - |
| gluconate | - | + |
| propionate | - | - |
| malainate | - | - |
| succinate | - | + |
| malate | + | + |

According to the above taxonomical properties, strain H-804 is a bacterium having the characteristics of round edges, straight or slightly curved bacilli, Gram-positive, sporulating at 0.5 to 1.0 x 1.6 to 5.5 µm thermophillic bacterium, weak catalase and oxidase production, no motile and oxidative degradation of sugar ( glucose ). Comparing these taxonomical properties with " Bergey's Manual ", 8th Ed., 1974, " Manual of Medicinal Bacteriology ", 2nd Ed., 1974 and " Agriculture Handbook ", p. 427, " The genus Bacillus ", the present strain is characterized by spore formation and aerobic growth and so belongs to the genus Bacillus . Among strains belonging to genus Bacillus which are thermophilic and thermotolerant are ( a ) Bacillus subtilis, ( b ) Bacillus coagulance, ( c ) Bacillus liqueniformis , ( d ) Bacillus brevis and ( e ) Bacillus stearothermophilus. The present strain can grown at above 30°C, and therefore is either ( A ) Bacillus stearothermophilus or ( B ) Bacillus brevis. Comparison of these strains is as follows : [ +: positive, -: negative, d: different in strain ]

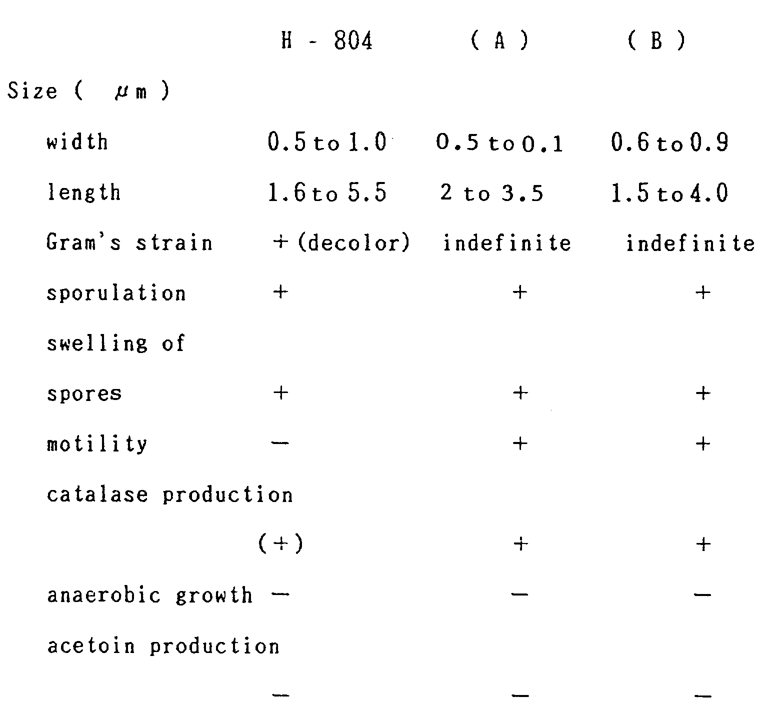

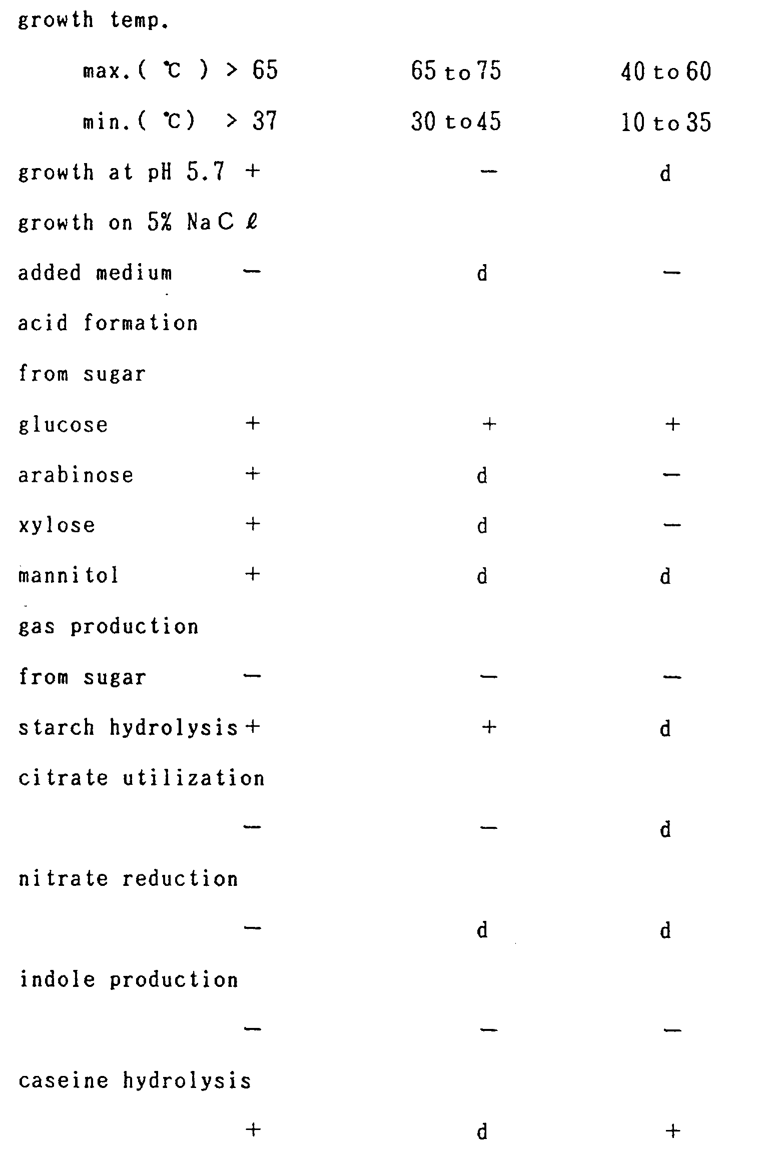

The properties of the present strain are quite similar to those of Bacillus stearothermophilus , and it is reported that a non-motile strain can easily be obtained. A comparison of the other properties suggest an identification of the present strain as Bacillus stearothermophilus. Thus the present strain is designated Bacillus stearothermophilus H-804. The strain has been deposited in the Fermentation Research Institute under the number FERM BP-1408.

In the present invention, the above strain is an example of NAD synthetase producing microorganisms belonging to genus Bacillus, but any strain which belongs to genus Bacillus and which produces the present NAD-synthetase can be used.

An artificial mutant can be prepared by isolating the DNA which bears the genetic code of the present NAD synthetase by recombinant DNA technology from the present NAD synthetase producing microorganisms, cloning the said DNA into another microorganism which does not produce the present NAD synthetase, and expressing the present NAD synthetase producing activity. An enzyme produced by such a mutant and an assay method using the same is included in the scope of the present invention.

The NAD-synthetase-producing microorganism belonging to the genus Bacillus may be cultured in a conventional medium for enzyme production. Cultivation is carried out in liquid or solid culture; submerged aeration culture is preferred for industrial production.

The nutrient sources of the medium may be conventional media for microorganism cultivation. The carbon sources are assimilable carbon compounds, for example glucose, sucrose, lactose, maltose, starch, dextrin, molasses or glycerin. The nitrogen sources are assimilable nitrogen sources such as corn steep liquor, soybean powder, cotton seed powder, wheat gluten, peptone, meat extract, yeast extract or casein hydrolyzate. Salts such as magnesium, potassium sodium, zinc, iron or manganese salts and phosphates or halides can be used.

The culturing temperature for Bacillus stearothermophilus H-804 can be chosen for its growth and the production of the enzyme. The temperature is, for example, from 48 to 70 °C, preferably from 55 to 60 °C. The culturing time can be varied depending on the culturing conditions, and is generally from 10 to 20 hours. The cultivation should naturally be stopped upon the maximum production of the enzyme. The aeration agitation is usually from 200 to 400 r.p.m.

Since the enzyme is an endo-enzyme, the cultured cells are collected by filtration or centrifugation, and the collected cells are mechanically disrupted by ultrasonication. French pressing or glass-beads treatment, or enzymatic digestion by a lysozyme, with the addition, if necessary, of surface active agents such as Triton X-100 ( trade name ) or Adekatol SO-120 (trdae name ) may be carried out.

The enzyme solution is, with or without concentration, subjected to salting-out by adding soluble salts such as ammonium sulfate, or treated by adding a water-miscible organic solvent such as methanol, ethanol, acetone or isopropanol to precipitate the enzyme. The precipitate is dissolved in water or a buffer solution, dialyzed if necessary, and chromatographed by an ion exchange resin such as DEAE-Sephadex, DEAE-Sepharose, carboxymethyl cellulose, carboxymethyl Sepharose or carboxymethyl Sephadex, or by gel-filtration using a molecular seive such as Sephadex G-200, Sephadex CL-6B OR Sephacryl S-200 ( trade names ). If required stabilizing agents may be added and lyophilization carried out to prepare a purified enzyme.

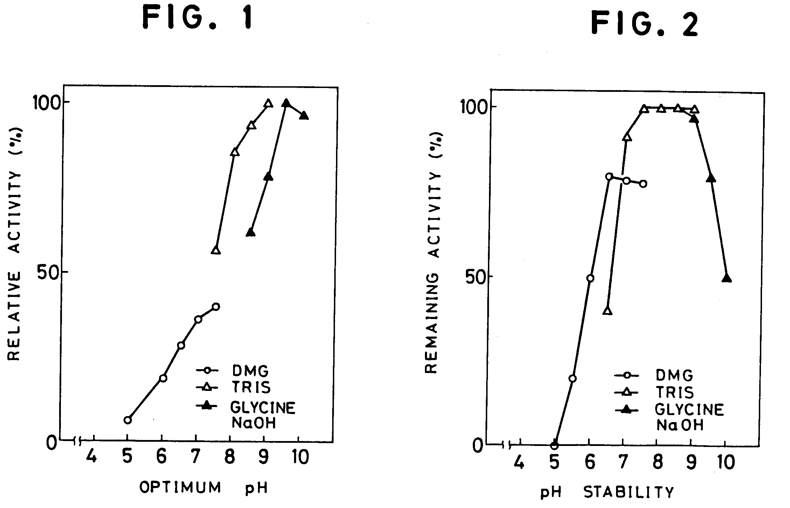

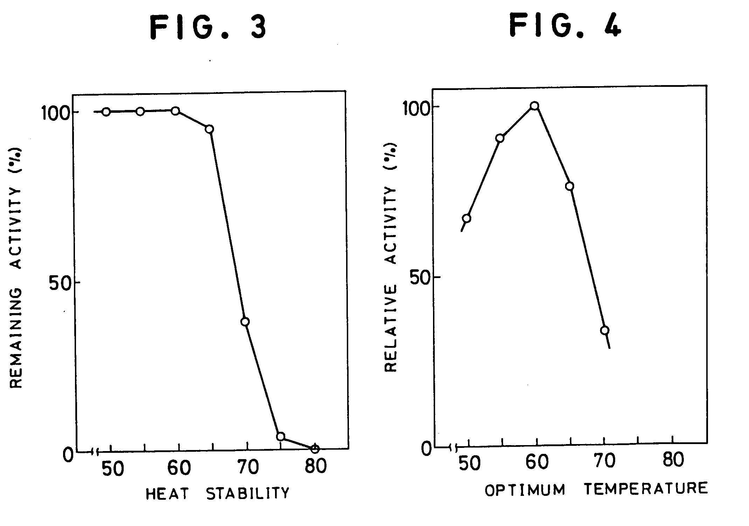

The biochemical properties of the above NAD synthetase are as follows :
(1) Molecular weight : approximately 50,000 [gel filtration by polyvinyl-gel( trade name GPC 3,000 SW: Toyo Soda Co., ), using a column ( 7.5 mm ID x 60 cm ). Standard proteins : aldorase (rabbit muscle, N.W. 150,000 ), bovine serum albumin ( M.W. 67,000 ) , avoalbumin ( egg : M.W. 45,000 ) and cytochrome C ( horse meat, M₂ W. 13,000 )]
(2) Isoelectroric point: approximately pH 5.3 ( electrophoresis using carrier-Ampholite, using a column ( 24 x 30 cm LKB Co. ), 700V, 48 hrs. cutting each 2 cm, measured pH and activity ) Mg⁺⁺ or + Mn⁺⁺
(3) Activity : ATP+deamide-NAD + NH₃ → ATP+PPi + NAD
(4) Substrate specificity :
   NH₃ including ammonium ions.
   In any assay method for enzyme activity hereinafter described, ( NH₄ )₂SO₄ 25 mM may be replaced by L-valine, L-homosirine, L-serine, L-alanine, L-methine, L-tyrosine, L-threonine, L-leucine, L-isolucine, L-arginine, L-phenylalanine, L-histidine, L-asparagine or L-glutamine, and the enzymatic activity measured. The relative activity of the enzyme on these amino acids is 0.0 when that on ( NH₄ )₂SO₄ is 100. Therefore the present enzyme can only utilize ammonia including ammonium ions and does not utilize at least the abovementioned amino acids.
(5) Optimum pH :
   Reaction medium I, identified hereinafter under the assay method for enzymatic activity, is mixed with dimethylglutarate-NaOH buffer ( pH 5.0 to 7.0 ), Tris-HCℓ buffer ( pH 6.5 to 9.0 ) and glycine-NaOH buffer ( pH 8.5 to 10.0 ). The enzyme activity is measured after stopping the enzyme action by heating at 100 °C for 10 minutes. As shown in Fig. 1, the optimum pH is from 8.5 to 10.0.
(6) pH-stability :
   The enzyme is dissolved in 50 mM dimethylglutarate-NaOH buffer ( pH 5.0 to 7.0 ), Tris-HCℓ buffer ( pH 6.5 to 9.0 ), or glycine-NaOH buffer ( pH 8.5 to 10.0 ), and the solution is incubated at 60 °C for 15 minutes. The remaining enzyme activity is measured by an assay. The results are shown in Fig. 2; the enzyme is stable at a pH of from 7.5 to 9.0.
(7) Heat stability :
   The enzyme dissolved in 50 mM Tris-HCℓ buffer ( pH 8.0 ) is held at various temperature for 15 minutes each and the remaining enzyme activity is measured. The results are shown in Fig. 3; the enzyme is stable at least below 60°C.
(8) optimum temperature :
   The enzyme mixed with reaction medium I hereinafter defined is incubated at temperatures of 50°C, 55°C, 60°C, 65°C and 70 °C for 10 minutes, immediately cooled thereafter, reaction medium II added thereto at 37 °C , and the enzyme activity measured. The results are shown in Fig. 4; the optimum temperature is approximately 60°C.
(9) Effect of enzyme activator and inhibitor :
   In the enzyme activity assay, each metallic ion ( 5 mM ), EDTA ( 20 mM ) or each surface active agent ( 0.1 % ) in Table 1 is added separately to the reaction medium I, and the enzyme activity is measured. The results are shown in Table 1. The enzyme is inhibited by Ni-iron ( NiCℓ₂, 5 mM ) and no activity is observed in EDTA ( 20 mM ).
   Furthermore, MgCℓ₂ ( 5 mM ) is replaced by MnCℓ₂ ( 3 mM ) in the reagent identified in the enzyme assay method hereinafter defined. The activity of the enzyme is increased to 150 % in the presence of MnCℓ₂ ( 3 mM ) as compared with MgCℓ₂ ( 5 mM ). 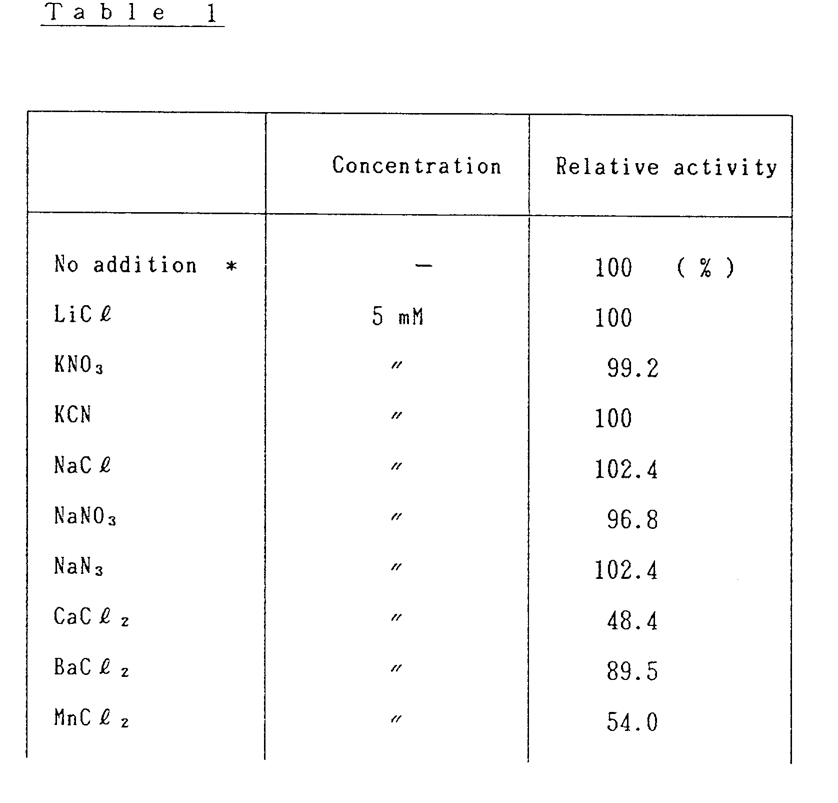 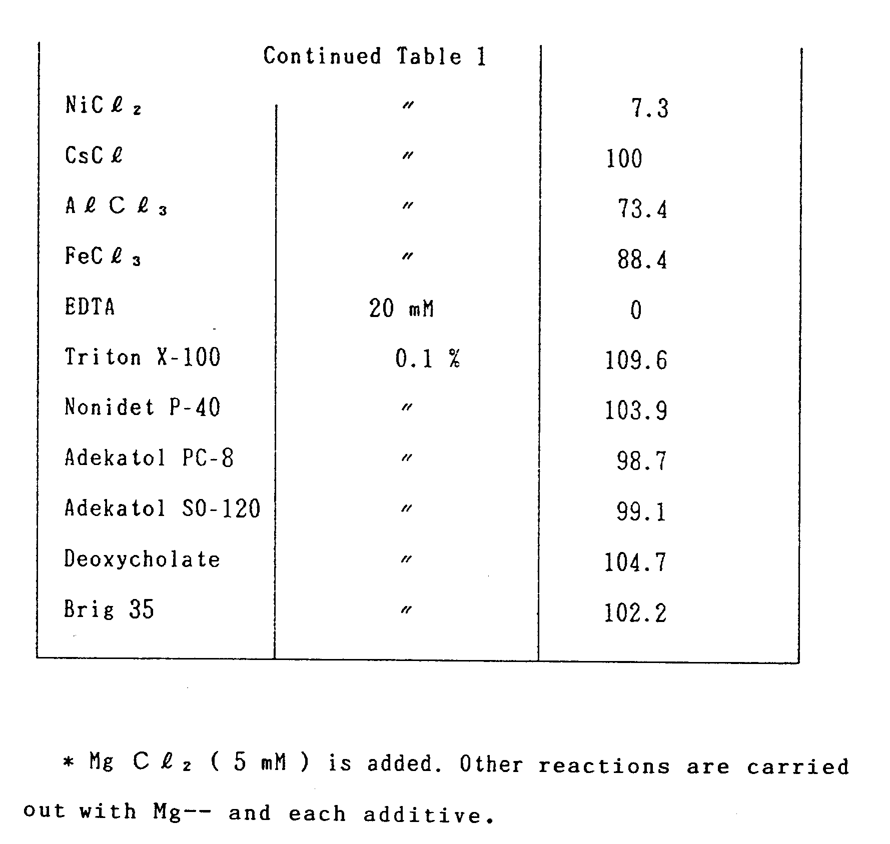
(10) Assay method of enzyme activity :
   Enzyme activity assay :
   Reaction medium I
   50 mM Tris-HCℓ buffer pH 8.0
   1 mM K Cℓ
   5 mM MgCℓ₂
   0.05 % bovine serum albumin
   2 mM ATP
   0.5 mM deamide-NAD
   25 mM ( NH₄ )₂ SO₄
   Reaction medium II
   50 mM Tris-HCℓ buffer pH 8.0
   10 U diaphorase ( Toyo Jozo Co. from genus Bacillus)
   3 % ethanol
   10 U alcohol dehydrogenase/mℓ ( Toyo Jozo, yeast )
   0.025 % NTB ( nitrotetrazolium blue )
   0.1 % Triton X-100
   10 mM EDTA
Reaction medium I ( 0.3 mℓ ) in a test tube is pre-incubated at 37 °C, and the enzyme solution ( 5 µℓ ) is added thereto. The mixture is then incubated at 37 °C for exactly 10 minutes.

Reaction medium II ( 0.8 mℓ ) is added thereto to stop the reaction and simultaneously to start the cycling reaction at 37°C for exactly 5 minutes. After stopping the cycling reaction by adding 0.1 N-HCℓ ( 2.0 mℓ ), the absorbency at 550 nm is measured to calculate the enzyme activity. The enzyme activity is calculated by the following equation : 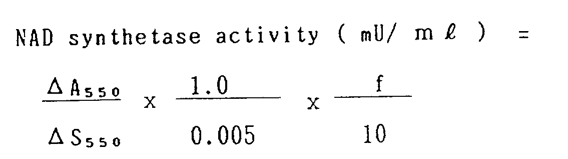
wherein
- ΔA:: absorbency of specimen,
- ΔS:: absorbency of standard solution ( 0.1 mM NAD ),
- 0.005:: specimen volume ( mℓ )
- 10 :: reaction time,
- f :: dilution ration.

An assay specimen which can be assayed by the NAD synthetase of the present invention can be a specimen containing at least ATP, deamide-NAD or ammonia including ammonium ions, for example a specimen previously containing any one of the components or a specimen containing generated or consumed components.

A preferred example of the enzyme reaction system is a reaction system which consumes or generates ATP, deamide-NAD or NH₃ including ammonium ions, without the coenzyme NAD and NADH.

Not only the reaction mixture containing ATP or NH₃, which is consumed or generated in the enzymatic reaction system, but also the reaction mixture for measuring the enzymatic activity which is used in the enzymatic reaction system, consumed substrate or generated product, can be used as a specimen to be assayed.

In these enzymatic reaction systems, ATP or NH₃ is assayed to determine the enzymatic activity in the said enzymatic reaction or measure the amount of any one of the components thereof . A substance other than the components to be assayed is added at a constant rate as a reagent. The amount of the specimen or reagent can be varied depending on the objects and conditions.

The assay method using the NAD synthetase can take the form, for example, of an end-point assay method, a rate assay method or a dry-chemical method ( film method or immobilized solid ).

The method is useful for the assay of any one of ATP, deamide-NAD or ammonia including ammonia in the form of ammonium ions. Ammonia including ammonium ions can be assayed without affecting the amino acids in a specimen. Furthermore, the NAD synthetase of the present invention is heat stable.

The following Examples further illustrate the present invention.

### Example 1

A liquid medium ( pH 7.6, 40 l ) consisting of 1% peptone, 0.5% glucose, 0.05% NaCℓ and 0.05% MgSO₄ · 7H₂O in a 50 ℓ jar fermenter was sterilized at 120 °C for 20 minutes. A previously-cultured Bacillus stearothermophilus H-804 seed-culture medium of the same composition ( 200 ml ) was inoculated therein and the mixture was cultured at 60°C for 10 hours with an aeration of 40 ℓ/min and an agitation of 150 r.p.m. After cultivation, the cells were collected by centrifugation, suspended in 10 mM tris-HCℓ ( pH 8.0, 500 mℓ ) containing 0.1 % lysozyme, and the medium was incubated at 37°C for 30 mins to lyse the cells. The lysed solution was centrifuged at 5,000 r.p.m. for 10 mins to obtain a supernatant solution ( 450 mℓ .) Ammonium sulfate was added thereto to fractionate the solution ( 0.5 to 0.71 saturation ) and the obtained preciptiate , dissolved in 10 mM Tris-HCℓ buffer ( 50mℓ , 21 U), was dialyzed against the same buffer ( 5 ℓ ) The preciptated insolubles were removed by centrifugation ( 15,000 r.p.m., 10 min ). The supernatant solution ( 20 U ) was charged on a column ( 2.5 x 5 cm ) of DEAE-Sepharose CL-6B buffered with 10 mM Tris-HCℓ buffer ( pH 8.0 ) and eluted with a gradient of 0 to 0.5 M NaCℓ. The fractions eluting with 0.25 to 0.3 M NaCℓ were collected ( 80 mℓ, 16.5 U ), concentrated by ultra-filtration using a CF-25 membrane ( Amicon Co. centriflow membrane cone ), chromatographed with Sephadex G-100 ( 3.6 x 80 cm ) and the active fractions collected to obtain the purified solution ( 5 mℓ, 14 U).

## Claims

1. A NAD synthetase having the following properties:
(1) Action:
at least catalyses in the presence of Mg⁺⁺ or Mn⁺⁺ ion the reaction (a): 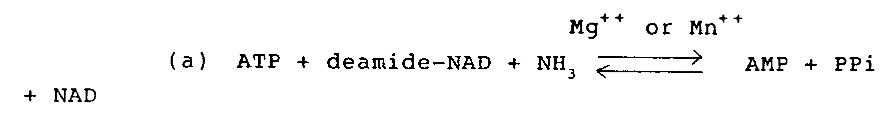 and does not catalyse in the presence of Mg⁺⁺ ion the reaction (b): 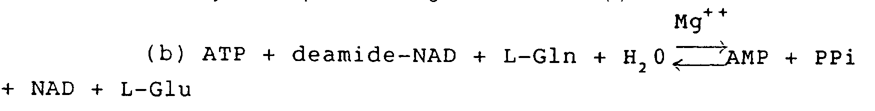
(2) Substrate specificity:
utilises ammonia, including ammonia in the form of an ammonium ion, as a substrate but does not utilise glutamine or asparagine as a substrate;
(3) optimum pH : pH 8.5. - 10.0;
(4) pH-stability : pH 7.5 - 9.0 (at 60°, 15 min);
(5) optimum temperature : approx. 60°C;
(6) heat stability : below 60°C (at pH 8.0, 15 min);
(7) isoelectric point: approx. pH 5.3; and
(8) molecular weight : 50,000 ± 5,000 (molecular sieve using polyvinyl gel).

2. A NAD synthetase according to claim 1 which is obtainable from Bacillus stearothermophilus H-804 (FERM BP-1408).

3. A process for the preparation of a NAD synthetase as defined in claim 1, which process comprises culturing Bacillus stearothermophilus H-804 (FERM BP-1408) or a mutant thereof which is capable of producing said NAD synthetase and isolating the NAD synthetase thus-produced.

4. Bacillus stearothermophilus H-804 (FERM BP-1408) or a mutant thereof which is capable of producing the NAD synthetase defined in claim 1.

5. A culture of Bacillus stearothermophilus H-804 (FERM BP-1408) or a mutant thereof which is capable of producing the NAD synthetase defined in claim 1, in a culture medium which comprises a source of assimilable carbon, a source of assimilable nitrogen and mineral salts and which is free of other microorganisms.

## Patentansprüche

1. NAD-Synthetase mit den folgenden Eigenschaften:
(1) Wirkung:
katalysiert mindestens in Gegenwart von Mg⁺⁺-Ionen oder Mn⁺⁺-Ionen die Reaktion (a): 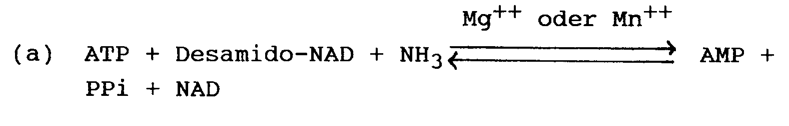 und katalysiert in Gegenwart von Mg⁺⁺-Ionen nicht die Reaktion (b): 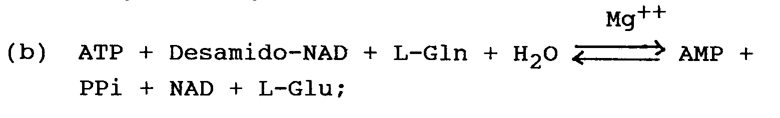
(2) Substratspezifität:
verwertet Ammoniak, einschließlich Ammoniak in Form eines Ammoniumions, als Substrat, aber verwertet nicht Glutamin oder Asparagin als Substrat;
(3) pH-Optimum: pH 8,5 - 10,0;
(4) pH-Stabilität: pH 7,5 - 9,0 (bei 60°C, 15 min);
(5) Temperaturoptimum: etwa 60°C;
(6) Hitzestabilität: unter 60°C (bei pH 8,0, 15 min);
(7) isoelektrischer Punkt: etwa pH 5,3; und
(8) Molekulargewicht: 50000 ± 5000 (Molekülsieb unter Verwendung eines Polyvinylgels).

2. NAD-Synthetase nach Anspruch 1, dadurch **gekennzeichnet**, daß sie aus Bacillus stearothermophilus H-804 (FERM BP-1408) erhältlich ist.

3. Verfahren zur Herstellung einer NAD-Synthetase nach Anspruch 1, dadurch **gekennzeichnet**, daß man Bacillus stearothermophilus H-804 (FERM BP-1408) oder eine Mutante davon mit der Fähigkeit, die NAD-Synthetase zu produzieren, züchtet und die so produzierte NAD-Synthetase isoliert.

4. Bacillus stearothermophilus H-804 (FERM BP-1408) oder eine Mutante davon mit der Fähigkeit zur Produktion der NAD-Synthetase nach Anspruch 1.

5. Bacillus stearothermophilus H-804 (FERM BP-1408)-Kultur oder eine Mutante davon mit der Fähigkeit zur Produktion der NAD-Synthetase nach Anspruch 1 in einem Kulturmedium, umfassend eine Quelle von assimilierbarem Kohlenstoff, eine Quelle von assimilierbarem Stickstoff und Mineralsalze, das frei von anderen Mikroorganismen ist.

## Revendications

1. Synthétase NAD ayant les propriétés suivantes :
(1) Action :
catalyse au moins en présence de l'ion Mg⁺⁺ ou 5 Mn⁺⁺ la réaction (a): 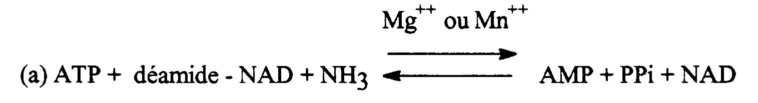 et ne catalyse pas en présence de l'ion Mg⁺⁺ la réaction (b) : 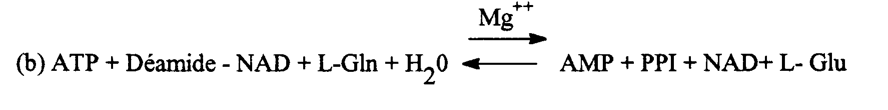
(2) Spécificité du substrat :
utilise l'ammoniaque, incluant l'ammoniaque sous la forme d'un ion ammonium, en tant que substrat mais n'utilise pas la glutamine ou l'asparagine en tant que substrat ;
(3) pH optimum : pH 8,5 - 10,0 ;
(4) stabilité - pH : pH 7,5 - 9,0 (à 60°C, 15 minutes) ;
(5) température optimale : approximativement 60°C ;
(6) stabilité à la chaleur : inférieure à 60°C (à pH, 8,0, 15 - 20 minutes) ;
(7) point isoélectrique : approximativement pH 5,3 ; et
(8) poids moléculaire : 50000 ± 5 000 (tamis moléculaire utilisant un gel de polyvinyle).

2. Synthétase NAD selon la revendication 1 que l'on peut obtenir à partir de Bacillus stearothermophilus H-804 (FERM BP-1408).

3. Procédé pour la préparation d'une synthétase NAD telle que définie à la revendication 1, qui comprend la culture de Bacillus stearothermophilus H-804 (FERM BP-1408) ou un mutant de celui-ci et qui est capable de produire ladite synthétase NAD et l'isolation de la synthétase NAD ainsi produite.

4. Bacillus stearothermophilus H-804 (FERM BP-1408) ou un mutant de celui-ci qui est capable de produire la synthétase NAD définie à la revendication 1.

5. Culture de Baccilus stearothermophilus H-804 (FERM BP-1408) ou un mutant de celui-ci qui est capable de produire la synthétase NAD définie à la revendication 1, dans un milieu de culture qui comprend une source de carbone assimilable, une source d'azote assimilable et des sels minéraux et qui est libre d'autres microorganismes.
